(19) Europäisches Patentamt
European Patent Office
Office européen des brevets

(11) EP 3 519 547 B1

(12) # EUROPEAN PATENT SPECIFICATION

(45) Date of publication and mention of the grant of the patent:
**09.04.2025 Bulletin 2025/15**

(21) Application number: **17772080.2**

(22) Date of filing: **29.09.2017**

(51) International Patent Classification (IPC):
***C11D 3/38*** *(2006.01)* ***C11D 3/386*** *(2006.01)*
***C11D 17/00*** *(2006.01)* ***A23K 10/30*** *(2016.01)*
***A23K 20/189*** *(2016.01)*

(52) Cooperative Patent Classification (CPC):
**C11D 17/0039; A23K 10/30; A23K 20/189; C11D 3/381; C11D 3/38672**

(86) International application number:
**PCT/EP2017/074864**

(87) International publication number:
**WO 2018/060475 (05.04.2018 Gazette 2018/14)**

(54) **SPORE CONTAINING GRANULE**

SPORENHALTIGES GRANULAT

BACTÉRIE CONTENANT UN GRANULE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.09.2016 EP 16191404**

(43) Date of publication of application:
**07.08.2019 Bulletin 2019/32**

(73) Proprietor: **Novozymes A/S**
**2880 Bagsvaerd (DK)**

(72) Inventor: **BACH, Poul**
**2880 Bagsvaerd (DK)**

(74) Representative: **NZ EPO Representatives**
**Krogshoejvej 36**
**2880 Bagsvaerd (DK)**

(56) References cited:
**EP-A2- 0 356 240**
**WO-A1-02/068573**
**WO-A1-2006/034710**
**WO-A1-2006/121596**
**DE-C1- 4 428 834**
**US-A- 4 106 991**
**US-A1- 2006 247 150**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 519 547 B1

## Description

### FIELD OF THE INVENTION

The present invention relates to enzyme and spore containing granules for use in a variety of applications, such as detergents, food and feed.

### BACKGROUND

International patent application WO 2012/112718 describes the use of microorganisms to control malodor in cleaning machines and cleaning processes.

International patent application WO 2011/018509 discloses dehydrated microorganisms surrounded by a coating comprising at least 25% of hygroscopic salt. Preferred dehydrated microorganisms are lactic acid bacteria used as "probiotics" in foods and feeds. Nothing is mentioned about microbial spores.

WO 2006/121596 discloses a stable solid cleaning composition including a borate salt and spores, vegetative bacteria, fungi or enzyme, and to methods of using the composition.

EP 356 240 discloses a process for making particles containing biologically produced material in a matrix of polymeric material, where the biologically produced material can be plant extracts, enzymes, fungi, spores, bacteria, cells and antibiotics.

### SUMMARY OF THE INVENTION

In a first aspect, the present invention provides an enzyme granule comprising a core and a coating, wherein the core comprises an enzyme and the coating comprises one or more spore(s).

The invention further provides a method of preparing the granules and a (powder) detergent composition comprising the enzyme granules.

Other aspects and embodiments of the invention are apparent from the description and examples.

### DETAILED DESCRIPTION

The use of bacteria to control malodor in cleaning machines and cleaning processes is described in WO 2012/112718. The required amount of bacteria on a mass basis is very small compared to the typical amounts of other ingredients used in cleaning compositions, such as detergent powders, tablets or gels. The dosage needed is in the order of a few grams of bacteria or spores per ton of detergent powder. The minimum amount of a powder that under normal industrial conditions can be mixed into a detergent powder, and still get sufficiently well distributed, is of the order 0.5 to 1%, or 5 to 10 kg per ton. Consequently, it is both a technical and economical challenge to apply the technology. The present invention makes it possible to distribute small amounts of spores uniformly by using enzyme granules as a vehicle.

Not only is it a challenge to obtain a uniform distribution of small amounts of spores, but it is also often undesirable to introduce microorganisms into processing equipment used for enzyme formulation. The invention avoids this by making an enzyme-containing raw granule (the core) and subsequently applying a spore-containing coating. By carrying out the two steps in two different locations, the bacterial spores are not introduced into the enzyme processing facility.

Thus, the present invention solves both of the above-mentioned problems in an efficient way, both from a technical and economic as well as from an environmental point of view. The invention shows that spore forming bacteria can be co-granulated into a finished enzyme/microorganism product that uniformly delivers both the necessary amount of spores with good viability and at the same time deliver enzymes, which are to be used in the same application.

### Enzyme granule

An enzyme granule of the invention is a small particle comprising an enzyme and spore(s), preferably bacterial (endo)spore(s).

The granule is composed of a core, and one or more coatings (outer layers) surrounding the core.

The core comprises the enzyme and a coating comprises the spore(s).

Typically, the particle size, measured as equivalent spherical diameter (volume based average particle size), of the granule is 20-2000 μm, particularly 50-1500 μm, 100-1500 μm or 250-1200 μm. Preferably, the granule is (roughly) spherical.

Core

The core may include additional materials such as fillers, fibre materials (cellulose or synthetic fibres), stabilizing agents, solubilising agents, suspension agents, viscosity regulating agents, light spheres, plasticizers, salts, lubricants and fragrances.

The core may include binders, such as synthetic polymer, wax, fat, or carbohydrate.

The core may comprise a salt of a multivalent cation, a reducing agent, an antioxidant, a peroxide decomposing catalyst and/or an acidic buffer component, typically as a homogenous blend.

The core may consist of an inert particle with the enzyme absorbed into it, or applied onto the surface, *e.g.*, via fluid bed coating.

The core particle may have a diameter of 20-2000 μm, particularly 50-1500 μm, 100-1500 μm or 250-1200 μm.

Preparation of core

The core can be prepared by granulating a blend of the ingredients, *e.g.*, by a method comprising granulation techniques such as crystallization, precipitation, pan-coating, fluid bed coating, fluid bed agglomeration, rotary atomization, extrusion, prilling, spheronization, size reduction methods, drum granulation, and/or high shear granulation.

Methods for preparing the core can be found in Handbook of Powder Technology; Particle size enlargement by C. E. Capes; Volume 1; 1980; Elsevier. Preparation methods include known feed and granule formulation technologies, *e.g.*:

a) Spray dried products, wherein a liquid enzyme-containing solution is atomized in a spray drying tower to form small droplets which during their way down the drying tower dry to form an enzyme-containing particulate material. Very small particles can be produced this way (Michael S. Showell (editor); Powdered detergents; Surfactant Science Series; 1998; vol. 71; page 140-142; Marcel Dekker).

b) Layered products, wherein the enzyme is coated as a layer around a pre-formed inert core particle, wherein an enzyme-containing solution is atomized, typically in a fluid bed apparatus wherein the pre-formed core particles are fluidized, and the enzyme-containing solution adheres to the core particles and dries up to leave a layer of dry enzyme on the surface of the core particle. Particles of a desired size can be obtained this way if a useful core particle of the desired size can be found. This type of product is described in, *e.g.*, WO 97/23606

c) Absorbed core particles, wherein rather than coating the enzyme as a layer around the core, the enzyme is absorbed onto and/or into the surface of the core. Such a process is described in WO 97/39116.

d) Extrusion or pelletized products, wherein an enzyme-containing paste is pressed to pellets or under pressure is extruded through a small opening and cut into particles which are subsequently dried. Such particles usually have a considerable size because of the material in which the extrusion opening is made (usually a plate with bore holes) sets a limit on the allowable pressure drop over the extrusion opening. Also, very high extrusion pressures when using a small opening increase heat generation in the enzyme paste, which is harmful to the enzyme (see also Michael S. Showell (editor); Powdered detergents; Surfactant Science Series; 1998; vol. 71; page 140-142; Marcel Dekker).

e) Prilled products, wherein an enzyme-containing powder is suspended in molten wax and the suspension is sprayed, *e.g.*, through a rotating disk atomiser, into a cooling chamber where the droplets quickly solidify (Michael S. Showell (editor); Powdered detergents; Surfactant Science Series; 1998; vol. 71; page 140-142; Marcel Dekker). The product obtained is one wherein the enzyme is uniformly distributed throughout an inert material instead of being concentrated on its surface. Also US 4,016,040 and US 4,713,245 are documents relating to this technique

f) Mixer granulation products, wherein a liquid is added to a dry powder composition of, *e.g.*, conventional granulating components, the enzyme being introduced either via the liquid or the powder or both. The liquid and the powder are mixed and as the moisture of the liquid is absorbed in the dry powder, the components of the dry powder will start to adhere and agglomerate and particles will build up, forming granulates comprising the enzyme. Such a process is described in US 4,106,991 and related documents EP 170360, EP 304332, EP 304331, WO 90/09440 and WO 90/09428. In a particular product of this process wherein various high-shear mixers can be used as granulators, granulates consisting of enzyme as enzyme, fillers and binders etc. are mixed with cellulose fibres to reinforce the particles to give the so-called T-granulate. Reinforced particles, being more robust, release less enzymatic dust.

g) Size reduction, wherein the cores are produced by milling or crushing of larger particles, pellets, tablets, briquettes etc. containing the enzyme. The wanted core particle fraction is obtained by sieving the milled or crushed product. Over and undersized particles can be recycled. Size reduction is described in (Martin Rhodes (editor); Principles of Powder Technology; 1990; Chapter 10; John Wiley & Sons).

h) Fluid bed granulation. Fluid bed granulation involves suspending particulates in an air stream and spraying a liquid onto the fluidized particles via nozzles. Particles hit by spray droplets get wetted and become tacky. The tacky particles collide with other particles and adhere to them and form a granule.

i) The cores may be subjected to drying, such as in a fluid bed drier. Other known methods for drying granules in the

feed or detergent industry can be used by the skilled person. The drying preferably takes place at a product temperature of from 25 to 90°C. For some enzymes it is important the cores comprising the enzyme contain a low amount of water before coating. If water sensitive enzymes are coated before excessive water is removed, it will be trapped within the core and it may affect the activity of the enzyme negatively. After drying, the cores preferably contain 0.1-10 % w/w water.

Coating

The core of the enzyme granule is surrounded by at least one coating, *e.g.*, to improve the storage stability, to reduce dust formation during handling, or for coloring the granule. The coating(s) may include a salt coating, or other suitable coating materials such as polyethylene glycol (PEG), methyl hydroxy-propyl cellulose (MHPC) and polyvinyl alcohol (PVA). These coatings include the spore(s). Examples of enzyme granules with multiple coatings are shown in WO 93/07263 and WO 97/23606.

The coating may be applied in an amount of at least 0.1% by weight of the core, e.g., at least 0.5%, 1% or 5%. The amount may be at most 100%, 70%, 50%, 40% or 30%.

The coating is preferably at least 0.1 μm thick, particularly at least 0.5 μm, at least 1 μm or at least 5 μm. In a particular embodiment the thickness of the coating is below 100 μm. In a more particular embodiment the thickness of the coating is below 60 μm. In an even more particular embodiment the total thickness of the coating is below 40 μm.

The coating should encapsulate the core unit by forming a substantially continuous layer. A substantially continuous layer is to be understood as a coating having few or no holes, so that the core unit it is encapsulating/enclosing has few or none uncoated areas. The layer or coating should in particular be homogeneous in thickness.

The coating can further contain other materials as known in the art, *e.g.*, fillers, antisticking agents, pigments, dyes, plasticizers and/or binders, such as titanium dioxide, kaolin, calcium carbonate or talc.

A salt coating may comprise at least 60% by weight w/w of a salt, *e.g.*, at least 65%, at least 70%, at least 75%, at least 80%, at least 85%, at least 90%, at least 95% or at least 99% by weight w/w.

The salt may be added from a salt solution where the salt is completely dissolved or from a salt suspension wherein the fine particles is less than 50 μm, such as less than 10 μm or less than 5 μm.

The salt coating may comprise a single salt or a mixture of two or more salts. The salt may be water soluble, in particular having a solubility at least 0.1 grams in 100 g of water at 20°C, preferably at least 0.5 g per 100 g water, *e.g.,* at least 1 g per 100 g water, *e.g.,* at least 5 g per 100 g water.

The salt may be an inorganic salt, *e.g.*, salts of sulfate, sulfite, phosphate, phosphonate, nitrate, chloride or carbonate or salts of simple organic acids (less than 10 carbon atoms, *e.g.*, 6 or less carbon atoms) such as citrate, malonate or acetate. Examples of cations in these salts are alkali or earth alkali metal ions, the ammonium ion or metal ions of the first transition series, such as sodium, potassium, magnesium, calcium, zinc or aluminium. Examples of anions include chloride, bromide, iodide, sulfate, sulfite, bisulfite, thiosulfate, phosphate, monobasic phosphate, dibasic phosphate, hypophosphite, dihydrogen pyrophosphate, tetraborate, borate, carbonate, bicarbonate, metasilicate, citrate, malate, maleate, malonate, succinate, lactate, formate, acetate, butyrate, propionate, benzoate, tartrate, ascorbate or gluconate. In particular alkali- or earth alkali metal salts of sulfate, sulfite, phosphate, phosphonate, nitrate, chloride or carbonate or salts of simple organic acids such as citrate, malonate or acetate may be used.

The salt in the coating may have a constant humidity at 20°C above 60%, particularly above 70%, above 80% or above 85%, or it may be another hydrate form of such a salt (*e.g.*, anhydrate). The salt coating may be as described in WO 00/01793 or WO 2006/034710.

Specific examples of suitable salts are NaCl ($CH_{20°C}$=76%), $Na_2CO_3$ ($CH_{20°C}$=92%), $NaNO_3$ ($CH_{20°C}$=73%), $Na_2HPO_4$ ($CH_{20°C}$=95%), $Na_3PO_4$ ($CH_{25°C}$=92%), $NH_4Cl$ ($CH_{20°C}$ = 79.5%), $(NH_4)_2HPO_4$ ($CH_{20°C}$ = 93,0%), $NH_4H_2PO_4$ ($CH_{20°C}$ = 93.1%), $(NH_4)_2SO_4$ ($CH_{20°C}$=81.1%), KCl ($CH_{20°C}$=85%), $K_2HPO_4$ ($CH_{20°C}$=92%), $KH_2PO_4$ ($CH_{20°C}$=96.5%), $KNO_3$ ($CH_{20°C}$=93.5%), $Na_2SO_4$ ($CH_{20°C}$=93%), $K_2SO_4$ ($CH_{20°C}$=98%), $KHSO_4$ ($CH_{20°C}$=86%), $MgSO_4$ ($CH_{20°C}$=90%), $ZnSO_4$ ($CH_{20°C}$=90%) and sodium citrate ($CH_{25°C}$=86%). Other examples include $NaH_2PO_4$, $(NH_4)H_2PO_4$, $CuSO_4$, $Mg(NO_3)_2$ and magnesium acetate.

The salt may be in anhydrous form, or it may be a hydrated salt, i.e. a crystalline salt hydrate with bound water(s) of crystallization, such as described in WO 99/32595. Specific examples include anhydrous sodium sulfate ($Na_2SO_4$), anhydrous magnesium sulfate ($MgSO_4$), magnesium sulfate heptahydrate ($MgSO_4 \cdot 7H_2O$), zinc sulfate heptahydrate ($ZnSO_4 \cdot 7H_2O$), sodium phosphate dibasic heptahydrate ($Na_2HPO_4 \cdot 7H_2O$), magnesium nitrate hexahydrate ($Mg(NO_3)_2(6H_2O)$), sodium citrate dihydrate and magnesium acetate tetrahydrate.

Preferably the salt is applied as a solution of the salt, *e.g.*, using a fluid bed.

Spores

The enzyme granule of the invention comprises one or more spores. In an embodiment, the one or more spores

are one or more bacterial (endo)spores.

In a more particular embodiment, the one or more bacterial spores are derived from spore forming bacterial strains. Methods for producing stabilized microorganisms, and bacterial spores specifically, are known in the art. See Donnellan, J. E., Nags, E. H., and Levinson, H. S. (1964) "Chemically defined, synthetic media for sporulation and for germination and growth of Bacillus subtilis", Journal of BacteriologyI, 87(2):332-336; and Chen, Z., Li, Q., Liu, H. Yu, N., Xie, T., Yang, M., Shen, P., Chen, X. (2010) "Greater enhancement of Bacillus subtilis spore yields in submerged cultures by optimization of medium composition through statistical experimental designs.", Appl. Microbiol. Biotechnol., 85:1353-1360.

Examples of spore forming bacterial strains include strains from the genera *Acetonema, Alkalibacillus, Ammoniphilus, Amphibacillus, Anaerobacter, Anaerospora, Aneurinibacillus, Anoxybacillus, Bacillus, Brevibacillus, Caldanaerobacter, Caloramator, Caminicella, Cerasibacillus, Clostridium, Clostridiisalibacter, Cohnella, Dendrosporobacter, Desulfotomaculum, Desulfosporomusa, Desulfosporosinus, Desulfovirgula, Desulfunispora, Desulfurispora, Filifactor, Filobacillus, Gelria, Geobacillus, Geosporobacter, Gracilibacillus, Halonatronum, Heliobacterium, Heliophilum, Laceyella, Lentibacillus, Lysinibacillus, Mahella, Metabacterium, Moorella, Natroniella, Oceanobacillus, Orenia, Omithinibacillus, Oxalophagus, Oxobacter, Paenibacillus, Paraliobacillus, Pelospora, Pelotomaculum, Piscibacillus, Planifilum, Pontibacillus, Propionispora, Salinibacillus, Salsuginibacillus, Seinonella, Shimazuella, Sporacetigenium, Sporoanaerobacter, Sporobacter, Sporobacterium, Sporohalobacter, Sporolactobacillus, Sporomusa, Sporosarcina, Sporotalea, Sporotomaculum, Syntrophomonas, Syntrophospora, Tenuibacillus, Tepidibacter, Terribacillus, Thalassobacillus, Thermoacetogenium, Thermoactinomyces, Thermoalkalibacillus, Thermoanaerobacter, Thermoanaeromonas, Thermobacillus, Thermoflavimicrobium, Thermovenabulum, Tuberibacillus, Virgibacillus,* and/ or *Vulcanobacillus.*

In a particular embodiment, the one or more spore forming bacteria is a bacteria selected from the genera consisting of *Acetonema, Alkalibacillus, Ammoniphilus, Amphibacillus, Anaerobacter, Anaerospora, Aneurinibacillus, Anoxybacillus, Bacillus, Brevibacillus, Caldanaerobacter, Caloramator, Caminicella, Cerasibacillus, Clostridium, Clostridiisalibacter, Cohnella, Dendrosporobacter, Desulfotomaculum, Desulfosporomusa, Desulfosporosinus, Desulfovirgula, Desulfunispora, Desulfurispora, Filifactor, Filobacillus, Gelria, Geobacillus, Geosporobacter, Gracilibacillus, Halonatronum, Heliobacterium, Heliophilum, Laceyella, Lentibacillus, Lysinibacillus, Mahella, Metabacterium, Moorella, Natroniella, Oceanobacillus, Orenia, Omithinibacillus, Oxalophagus, Oxobacter, Paenibacillus, Paraliobacillus, Pelospora, Pelotomaculum, Piscibacillus, Planifilum, Pontibacillus, Propionispora, Salinibacillus, Salsuginibacillus, Seinonella, Shimazuella, Sporacetigenium, Sporoanaerobacter, Sporobacter, Sporobacterium, Sporohalobacter, Sporolactobacillus, Sporomusa, Sporosarcina, Sporotalea, Sporotomaculum, Syntrophomonas, Syntrophospora, Tenuibacillus, Tepidibacter, Terribacillus, Thalassobacillus, Thermoacetogenium, Thermoactinomyces, Thermoalkalibacillus, Thermoanaerobacter, Thermoanaeromonas, Thermobacillus, Thermoflavimicrobium, Thermovenabulum, Tuberibacillus, Virgibacillus, Vulcanobacillus,* and combinations thereof.

In another embodiment, the one or more bacterial strains is a strain of *Bacillus spp., e.g., Bacillus alcalophilus, Bacillus alvei, Bacillus aminovorans, Bacillus amyloliquefaciens, Bacillus aneurinolyticus, Bacillus aquaemaris, Bacillus atrophaeus, Bacillus boroniphilius, Bacillus brevis, Bacillus caldolyticus, Bacillus centrosporus, Bacillus cereus, Bacillus circulans, Bacillus coagulans, Bacillus firmus, Bacillus flavothermus, Bacillus fusiformis, Bacillus globigii, Bacillus infernus, Bacillus larvae, Bacillus laterosporus, Bacillus lentus, Bacillus licheniformis, Bacillus megaterium, Bacillus, mesentericus, Bacillus mucilaginosus, Bacillus mycoides, Bacillus natto, Bacillus pantothenticus, Bacillus polymyxa, Bacillus pseudoanthracis, Bacillus pumilus, Bacillus schlegelii, Bacillus sphaericus, Bacillus sporothermodurans, Bacillus stearothermophillus, Bacillus subtilis, Bacillus thermoglucosidasius, Bacillus thuringiensis, Bacillus vulgatis, Bacillus weihenstephanensis,* and combinations thereof.

In another embodiment, the one or more bacterial strains is a strain of *Brevibacillus spp., e.g., Brevibacillus brevis*; *Brevibacillus formosus; Brevibacillus laterosporus;* or *Brevibacillus parabrevis,* and combinations thereof.

In another embodiment, the one or more bacterial strains is a strain of *Paenibacillus spp., e.g., Paenibacillus alvei; Paenibacillus amylolyticus; Paenibacillus azotofixans; Paenibacillus cookii; Paenibacillus macerans; Paenibacillus polymyxa;* or *Paenibacillus validus,* and combinations thereof.

In a more particular embodiment, the one or more bacterial strains is a strain of *Bacillus* selected from the group consisting of *Bacillus pumilus* strain NRRL B-50016 (from Novozymes Biologicals, Inc., USA); *Bacillus amyloliquefaciens* strain NRRL B-50017 (from Novozymes Biologicals, Inc., USA); *Bacillus amyloliquefaciens* strain PTA-7792 (previously classified as *Bacillus atrophaeus*) (from Novozymes Biologicals, Inc., USA); *Bacillus amyloliquefaciens* strain PTA-7543 (previously classified as *Bacillus atrophaeus*) (from Novozymes Biologicals, Inc., USA); *Bacillus amyloliquefaciens* strain NRRL B-50018 (from Novozymes Biologicals, Inc., USA); *Bacillus amyloliquefaciens* strain PTA-7541 (from Novozymes Biologicals, Inc., USA); *Bacillus amyloliquefaciens* strain PTA-7544 (from Novozymes Biologicals, Inc., USA); *Bacillus amyloliquefaciens* strain PTA-7545 (from Novozymes Biologicals, Inc., USA); *Bacillus amyloliquefaciens* strain PTA-7546 (from Novozymes Biologicals, Inc., USA); *Bacillus subtilis* strain PTA-7547 (from Novozymes Biologicals, Inc., USA); *Bacillus amyloliquefaciens* strain PTA-7549 (from Novozymes Biologicals, Inc., USA); *Bacillus amyloliquefaciens* strain PTA-7793 (from Novozymes Biologicals, Inc., USA); *Bacillus amyloliquefaciens* strain PTA-7790 (from Novozymes

Biologicals, Inc., USA); *Bacillus amyloliquefaciens* strain PTA-7791 (from Novozymes Biologicals, Inc., USA); *Bacillus subtilis* strain NRRL B-50136 (also known as DA-33R, ATCC accession No. 55406) (from Novozymes Biologicals, Inc., USA); *Bacillus amyloliquefaciens* strain NRRL B-50141 (from Novozymes Biologicals, Inc., USA); *Bacillus amyloliquefaciens* strain NRRL B-50399 (from Novozymes Biologicals, Inc., USA); *Bacillus licheniformis* strain NRRL B-50014 (from Novozymes Biologicals, Inc., USA); *Bacillus licheniformis* strain NRRL B-50015 (from Novozymes Biologicals, Inc., USA); *Bacillus amyloliquefaciens* strain NRRL B-50607 (from Novozymes Biologicals, Inc., USA); *Bacillus subtilis* strain NRRL B-50147 (also known as 300R) (from Novozymes Biologicals, Inc., USA); *Bacillus amyloliquefaciens* strain NRRL B-50150 (from Novozymes Biologicals, Inc., USA); *Bacillus amyloliquefaciens* strain NRRL B-50154 (from Novozymes Biologicals, Inc., USA); *Bacillus megaterium* PTA-3142 (from Novozymes Biologicals, Inc., USA); *Bacillus amyloliquefaciens* strain ATCC accession No. 55405 (also known as 300) (from Novozymes Biologicals, Inc., USA); *Bacillus amyloliquefaciens* strain ATCC accession No. 55407 (also known as PMX) (from Novozymes Biologicals, Inc., *USA);Bacillus pumilus* NRRL B-50398 (also known as ATCC 700385, PMX-1, and NRRL B-50255) (from Novozymes Biologicals, Inc., USA); *Bacillus cereus* ATCC accession No. 700386 (from Novozymes Biologicals, Inc., USA); *Bacillus thuringiensis* ATCC accession No. 700387 (from Novozymes Biologicals, Inc., *USA)Bacillus amyloliquefaciens* FZB24 (*e.g.,* isolates NRRL B-50304 and NRRL B-50349 TAEGRO® from Novozymes Biologicals, Inc., USA), *Bacillus subtilis* (*e.g.,* isolate NRRL B-21661 in RHAPSODY®, SERENADE® MAX and SERENADE® ASO from Bayer CropScience, Gustafson), *Bacillus pumilus* (*e.g.,* isolate NRRL B-50349 from Bayer CropScience, Gustafson), *Bacillus amyloliquefaciens* TrigoCor (also known as "TrigoCor 1448"; *e.g.*, isolate Embrapa Trigo Accession No. 144/88.4Lev, Cornell Accession No. Pma007BR-97, and ATCC accession No. 202152, from Cornell University, USA) and combinations thereof.

In still an even more particular embodiment, the one or more bacterial strains is a strain of *Bacillus amyloliquefaciens.* In an even more particular embodiment, the bacterial strain is *Bacillus amyloliquefaciens* strain PTA-7543 (previously classified as *Bacillus atrophaeus*) (from Novozymes Biologicals, Inc., USA), *Bacillus amyloliquefaciens* strain NRRL B-50154 (from Novozymes Biologicals, Inc., USA), or combinations thereof. In one embodiment, the bacterial strain is *Bacillus amyloliquefaciens* strain PTA-7543 (previously classified as *Bacillus atrophaeus*) (from Novozymes Biologicals, Inc., USA). In another embodiment the bacterial strain is *Bacillus amyloliquefaciens* strain NRRL B-50154 (from Novozymes Biologicals, Inc., USA).

In a particular embodiment, the one or more bacterial strains will be present in a quantity between $1x10^2$ and $1x10^{12}$ CFU/g of the granule, particularly $1x10^4$ and $1x10^{11}$ CFU/g of the granule, and more particularly $1x10^5$ and $5x10^{10}$ CFU/g of the granule. In a more particular embodiment the one or more bacterial strains will be present in a quantity between $1x10^6$ and $1x10^{10}$ CFU/g of the granule.

The fermentation of the one or more bacterial strains may be conducted using conventional fermentation processes, such as, aerobic liquid-culture techniques, shake flask cultivation, and small-scale or large-scale fermentation (*e.g.*, continuous, batch, fed-batch, solid state fermentation, etc.) in laboratory or industrial fermentors, and such processes are well known in the art. Notwithstanding the production process used to produce the one or more bacterial strains, the one or more bacterial strains may be used directly from the culture medium or subject to purification and/or further processing steps (*e.g.*, a drying process).

Following fermentation, the one or more bacterial strains may be recovered using conventional techniques (*e.g.*, by filtration, centrifugation, etc.). The one or more bacterial strains may alternatively be dried (*e.g.*, air-drying, freeze drying, or spray drying to a low moisture level, and storing at a suitable temperature, *e.g.*, room temperature).

## Enzyme

The enzyme included in the enzyme granule according to the invention, does not include any cytoplasmic enzyme in the spore(s). Thus, the spore(s) may contain intracellular enzymes, but these enzymes are not considered an enzyme according to the invention.

The enzyme granule as well as the detergent composition includes one or more enzymes, in particular enzymes suitable for including in laundry or dishwash detergents (detergent enzymes), such as a protease (e.g., subtilisin or metalloprotease), lipase, cutinase, amylase, carbohydrase, cellulase, pectinase, mannanase, arabinase, galactanase, xanthanase (EC 4.2.2.12), xylanase, DNase, perhydrolase, oxidoreductase (e.g., laccase, peroxidase, peroxygenase and/or haloperoxidase).

Preferred detergent enzymes are protease (e.g., subtilisin or metalloprotease), lipase, amylase, lyase, cellulase, pectinase, mannanase, DNase, perhydrolase, and oxidoreductases (e.g., laccase, peroxidase, peroxygenase and/or haloperoxidase); or combinations thereof. Particularly preferred detergent enzymes are protease (e.g., subtilisin or metalloprotease), lipase, amylase, cellulase, pectinase, and mannanase; or combinations thereof.

The enzyme may be a naturally occurring enzyme of bacterial or fungal origin, or it may be a variant derived from one or more naturally occurring enzymes by gene shuffling and/or by substituting, deleting or inserting one or more amino acids. Chemically modified or protein engineered mutants are included.

The enzyme granule may contain more than 0.5% w/w and less than 50% w/w active enzyme protein; preferably

more than 0.6% w/w and less than 40% w/w active enzyme protein; more preferably more than 0.75% w/w and less than 30% w/w active enzyme protein; and most preferably more than 1% w/w and less than 25% w/w active enzyme protein.

Proteases

The proteases for use in the present invention are serine proteases, such as subtilisins, metalloproteases and/or trypsin-like proteases. Preferably, the proteases are subtilisins or metalloproteases; more preferably, the proteases are subtilisins.

A serine protease is an enzyme which catalyzes the hydrolysis of peptide bonds, and in which there is an essential serine residue at the active site (White, Handler and Smith, 1973 "Principles of Biochemistry," Fifth Edition, McGraw-Hill Book Company, NY, pp. 271-272). Subtilisins include, preferably consist of, the I-S1 and I-S2 sub-groups as defined by Siezen et al., Protein Engng. 4 (1991) 719-737; and Siezen et al., Protein Science 6 (1997) 501-523. Because of the highly conserved structure of the active site of serine proteases, the subtilisin according to the invention may be functionally equivalent to the proposed sub-group designated subtilase by Siezen et al. (supra).

The subtilisin may be of animal, vegetable or microbial origin, including chemically or genetically modified mutants (protein engineered variants), preferably an alkaline microbial subtilisin. Examples of subtilisins are those derived from Bacillus, e.g., subtilisin Novo, subtilisin Carlsberg, subtilisin BPN', subtilisin 309, subtilisin 147 and subtilisin 168 (described in WO 89/06279) and Protease PD138 (WO 93/18140). Examples are described in WO 98/020115, WO 01/44452, WO 01/58275, WO 01/58276, WO 03/006602 and WO 04/099401.

Other examples of useful proteases are the variants described in WO92/19729, WO96/034946, WO98/20115, WO98/20116, WO99/011768, WO01/44452, WO03/006602, WO04/03186, WO04/041979, WO07/006305, WO11/036263, WO11/036264, especially the variants with substitutions in one or more of the following positions: 3, 4, 9, 15, 27, 36, 43, 57, 61, 62, 68, 76, 87, 95, 96, 97, 98, 99, 100, 101, 102, 103, 104, 106, 118, 120, 123, 128, 129, 130, 156, 158, 160, 161, 167, 170, 182, 185, 188, 191, 194, 195, 199, 204, 205, 206, 209, 212, 217, 218, 224, 232, 235, 236, 245, 248, 252, 261, 262, 274 and 275, using the BPN' numbering.

The protease may comprise a substitution at one or more positions corresponding to positions 171, 173, 175, 179, or 180 of SEQ ID NO: 3 of WO 2004/067737.

More preferred the protease variants may comprise one or more of the following substitutions: S3T, V4I, S9R, S9E, A15T, K27R, *36D, N43R, G61E, G61D, N62D, N62E, V68A, N76D, N87S,R, *97E, A98S, S99G,S99D, S99A, S99AD, S101E, S101D, S101G, S101M, S101N, S101R, S101H, S103A, V104I, V104Y, V104N, S106A, G118V, G118R, H120D, H120N, N123S, S128L, P129Q, S130A, S156D, A158E, G160D, G160P, S161E, Y167A, R170S, Q182E, N185E, S188E, Q191N, A194P, G195E, V199M, N204D, V205I, Y209W, S212G, L217Q, L217D, N218D, N218S, A232V, K235L, Q236H, Q245R, N252K, N261W, N261D, N261E, L262E, L262D T274A, R275H (using BPN' numbering).

Examples of commercially available proteases include those sold under the trade names Alcalase™, Relase™, Relase™ Ultra, Savinase™, Savinase™ Ultra, Duralase™, Durazym™, Everlase™, Primase™, Polarzyme™, Kannase™, Liquanase™, Liquanase™ Ultra, Ovozyme™, Coronase™, Coronase™ Ultra, Blaze™, Blaze Evity™ 100T, Blaze Evity™ 125T, Blaze Evity™ 150T, Neutrase™, and Esperase™ (Novozymes A/S); those sold under the tradename Maxatase™, Maxacal™, Puramax™, FN2™, FN3™, FN4™, Excellase™, Maxapem™, Purafect Ox™, Purafect OxP™, Effectenz™ P1050, Effectenz™ P1060, Excellenz™ P1000, Excellenz™ P1250, Eraser™, Preferenz™ P100, Purafect Prime™, Preferenz™ P110, Effectenz™ P1000, Purafect™, Effectenz™ P2000, Purafast™, Properase™, Opticlean™ and Optimase™ (Genencor/Danisco/DuPont); Axapem™ (Gist-Brocases N.V.); BLAP (sequence shown in Figure 29 of US5352604) and variants hereof (Henkel AG); and KAP (Bacillus alkalophilus subtilisin) from Kao.

Lyases

The lyase may be a pectate lyase derived from Bacillus, particularly B. licherniformis or B. agaradhaerens, or a variant derived of any of these, e.g. as described in US 6124127, WO 99/027083, WO 99/027084, WO 02/006442, WO 02/092741, WO 03/095638, Commercially available pectate lyases are XPect™; Pectawash™ and Pectaway™ (Novozymes A/S).

Mannanase

The mannanase may be an alkaline mannanase of Family 5 or 26. It may be a wild-type from Bacillus or Humicola, particularly B. agaradhaerens, B. licheniformis, B. halodurans, B. clausii, or H. insolens. Suitable mannanases are described in WO 99/064619. A commercially available mannanase is Mannaway™ (Novozymes A/S).

Cellulases

Suitable cellulases include those of bacterial or fungal origin. Chemically modified or protein engineered mutants are included. Suitable cellulases include cellulases from the genera Bacillus, Pseudomonas, Humicola, Fusarium, Thielavia, Acremonium, e.g., the fungal cellulases produced from Humicola insolens, Myceliophthora thermophila and Fusarium oxysporum disclosed in US 4,435,307, US 5,648,263, US 5,691,178, US 5,776,757 and WO 89/09259.

Especially suitable cellulases are the alkaline or neutral cellulases having color care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme™, and Carezyme™ (Novozymes A/S), Clazinase™, and Puradax HA™ (DuPont), and KAC-500(B)™ (Kao Corporation).

Lipases and Cutinases

Suitable lipases and cutinases include those of bacterial or fungal origin. Chemically modified or protein engineered mutants are included. Examples include lipase from Thermomyces, e.g., from T. lanuginosus (previously named Humicola lanuginosa) as described in EP 258 068 and EP 305 216, cutinase from Humicola, e.g., H. insolens as described in WO 96/13580, a Pseudomonas lipase, e.g., from P. alcaligenes or P. pseudoalcaligenes (EP 218 272), P. cepacia (EP 331 376), P. stutzeri (GB 1,372,034), P. fluorescens, Pseudomonas sp. strain SD 705 (WO 95/06720 and WO 96/27002), P. wisconsinensis (WO 96/12012), a Bacillus lipase, e.g., from B. subtilis (Dartois et al., 1993, Biochemica et Biophysica Acta, 1131: 253-360), B. stearothermophilus (JP 64/744992) or B. pumilus (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079, WO 97/07202, WO 00/060063, WO 2007/087508 and WO 2009/109500.

Preferred commercially available lipase enzymes include Lipolase™, Lipolase Ultra™, and Lipex™; Lecitase™, Lipolex™: Lipoclean™, Lipoprime™ (Novozymes A/S). Other commercially available lipases include Lumafast (DuPont); Lipomax (Gist-Brocades/DuPont) and Bacillus sp. lipase from Solvay.

Amylases

Suitable amylases ($\alpha$ and/or $\beta$) include those of bacterial or fungal origin. Chemically modified or protein engineered mutants are included. Amylases include, for example, $\alpha$ amylases obtained from Bacillus, e.g., a special strain of Bacillus licheniformis, described in more detail in GB 1,296,839.

Examples of suitable amylases include amylases having SEQ ID NO: 2 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181 and 182 and a substitution in position 193. Other amylases which are suitable are hybrid alpha-amylase comprising residues 1-33 of the alpha-amylase derived from B. amyloliquefaciens shown in SEQ ID NO: 6 of WO 2006/066594 and residues 36-483 of the B. licheniformis alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, I201, A209 and Q264. Most preferred variants of the hybrid alpha-amylase comprising residues 1-33 of the alpha-amylase derived from B. amyloliquefaciens shown in SEQ ID NO: 6 of WO 2006/066594 and residues 36-483 of SEQ ID NO: 4 are those having the substitutions:

M 197T;
H156Y+A181T+N190F+A209V+Q264S; or
G48A+T49I+G107A+H156Y+A181T+N190F+I201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476. More preferred variants are those having a deletion in positions 181 and 182 or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:

N128C+K178L+T182G+Y305R+G475K;
N128C+K178L+T182G+F202Y+Y305R+D319T+G475K;
S125A+N128C+K178L+T182G+Y305R+G475K; or
S125A+N128C+T131I+T165I+K178L+T182G+Y305R+G475K wherein the variants are C-terminally truncated and optionally further comprises a substitution at position 243 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Stainzyme™; Stainzyme Plus™; Duramyl™, Termamyl™, Termamyl Ultra™; Natalase™, Fungamyl™ and BAN™ (Novozymes A/S), Rapidase™ and Purastar™/Effectenz™, Powerase™ and Preferenz™ S100 (from DuPont).

Deoxyribonuclease (DNase)

Suitable deoxyribonucleases (DNases) are any enzyme that catalyzes the hydrolytic cleavage of phosphodiester linkages in the DNA backbone, thus degrading DNA. According to the invention, a DNase which is obtainable from a bacterium is preferred; in particular a DNase which is obtainable from a Bacillus is preferred; in particular a DNase which is obtainable from Bacillus subtilis or Bacillus licheniformis is preferred. Examples of such DNases are described in patent application WO 2011/098579 or in WO 2014/087011.

Perhydrolases

Suitable perhydrolases are capable of catalyzing a perhydrolysis reaction that results in the production of a peracid from a carboxylic acid ester (acyl) substrate in the presence of a source of peroxygen (e.g., hydrogen peroxide). While many enzymes perform this reaction at low levels, perhydrolases exhibit a high perhydrolysis:hydrolysis ratio, often greater than 1. Suitable perhydrolases may be of plant, bacterial or fungal origin. Chemically modified or protein engineered mutants are included.

Examples of useful perhydrolases include naturally occurring Mycobacterium perhydrolase enzymes, or variants thereof. An exemplary enzyme is derived from Mycobacterium smegmatis. Such enzyme, its enzymatic properties, its structure, and variants thereof, are described in WO 2005/056782, WO 2008/063400, US 2008/145353, and US

2007/167344.

Oxidases/peroxidases

Suitable oxidases and peroxidases (or oxidoreductases) include various sugar oxidases, laccases, peroxidases and haloperoxidases.

Suitable peroxidases include those comprised by the enzyme classification EC 1.11.1.7, as set out by the Nomenclature Committee of the International Union of Biochemistry and Molecular Biology (IUBMB), or any fragment derived therefrom, exhibiting peroxidase activity.

Suitable peroxidases include those of plant, bacterial or fungal origin. Chemically modified or protein engineered mutants are included. Examples of useful peroxidases include peroxidases from Coprinopsis, e.g., from C. cinerea (EP 179,486), and variants thereof as those described in WO 93/24618, WO 95/10602, and WO 98/15257.

A peroxidase for use in the invention also include a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (EC 1.11.1.10) catalyze formation of hypochlorite from chloride ions.

In an embodiment, the haloperoxidase is a chloroperoxidase. Preferably, the haloperoxidase is a vanadium haloperoxidase, i.e., a vanadate-containing haloperoxidase. In a preferred method of the present invention the vanadate-containing haloperoxidase is combined with a source of chloride ion.

Haloperoxidases have been isolated from many different fungi, in particular from the fungus group dematiaceous hyphomycetes, such as Caldariomyces, e.g., C. fumago, Alternaria, Curvularia, e.g., C. verruculosa and C. inaequalis, Drechslera, Ulocladium and Botrytis.

Haloperoxidases have also been isolated from bacteria such as Pseudomonas, e.g., P. pyrrocinia and Streptomyces, e.g., S. aureofaciens.

In an preferred embodiment, the haloperoxidase is derivable from Curvularia sp., in particular Curvularia verruculosa or Curvularia inaequalis, such as C. inaequalis CBS 102.42 as described in WO 95/27046; or C. verruculosa CBS 147.63 or C. verruculosa CBS 444.70 as described in WO 97/04102; or from Drechslera hartlebii as described in WO 01/79459, Dendryphiella salina as described in WO 01/79458, Phaeotrichoconis crotalarie as described in WO 01/79461, or Geniculosporium sp. as described in WO 01/79460.

An oxidase according to the invention include, in particular, any laccase enzyme comprised by the enzyme classification EC 1.10.3.2, or any fragment derived therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5).

Preferred laccase enzymes are enzymes of microbial origin. The enzymes may be derived from plants, bacteria or fungi (including filamentous fungi and yeasts).

Suitable examples from fungi include a laccase derivable from a strain of Aspergillus, Neurospora, e.g., N. crassa, Podospora, Botrytis, Collybia, Fomes, Lentinus, Pleurotus, Trametes, e.g., T. villosa and T. versicolor, Rhizoctonia, e.g., R. solani, Coprinopsis, e.g., C. cinerea, C. comatus, C. friesii, and C. plicatilis, Psathyrella, e.g., P. condelleana, Panaeolus, e.g., P. papilionaceus, Myceliophthora, e.g., M. thermophila, Schytalidium, e.g., S. thermophilum, Polyporus, e.g., P. pinsitus, Phlebia, e.g., P. radiata (WO 92/01046), or Coriolus, e.g., C. hirsutus (JP 2238885).

Suitable examples from bacteria include a laccase derivable from a strain of Bacillus.

A laccase derived from Coprinopsis or Myceliophthora is preferred; in particular a laccase derived from Coprinopsis cinerea, as disclosed in WO 97/08325; or from Myceliophthora thermophila, as disclosed in WO 95/33836.

Examples of other oxidases include, but are not limited to, amino acid oxidase, glucose oxidase, lactate oxidase, galactose oxidase, polyol oxidase (e.g., WO2008/051491), and aldose oxidase. Oxidases and their corresponding substrates may be used as hydrogen peroxide generating enzyme systems, and thus a source of hydrogen peroxide. Several enzymes, such as peroxidases, haloperoxidases and perhydrolases, require a source of hydrogen peroxide. By studying EC 1.1.3._, EC 1.2.3._, EC 1.4.3._, and EC 1.5.3._ or similar classes (under the International Union of Biochemistry), other examples of such combinations of oxidases and substrates are easily recognized by one skilled in the art.

Amino acid changes, as referenced above, may be of a minor nature, that is conservative amino acid substitutions or insertions that do not significantly affect the folding and/or activity of the protein; small deletions, typically of 1-30 amino acids; small amino or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino

acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for enzyme activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

The relatedness between two amino acid sequences is described by the parameter "sequence identity". For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment}).$$

**Detergent composition**

The enzyme granule of the invention may be added to and thus become a component of a detergent composition.

The detergent composition of the present invention may be formulated, for example, as a hand or machine laundry detergent composition including a laundry additive composition suitable for pre-treatment of stained fabrics and a rinse added fabric softener composition, or be formulated as a detergent composition for use in general household hard surface cleaning operations, or be formulated for hand or machine dishwashing operations.

In a specific aspect, the present invention provides a detergent additive comprising a granule of the present invention, as described herein.

In one embodiment, the invention is directed to detergent compositions comprising an enzyme granule of the present invention in combination with one or more additional cleaning composition components.

The detergent composition may be a solid (powder, granular) or a liquid detergent composition. A liquid detergent composition has a physical form, which is not solid (or gas). It may be a pourable liquid, a pourable gel or a non-pourable gel. It may be either isotropic or structured, preferably isotropic.

Liquids, including without limitation, alkanols, amines, diols, ethers and polyols may be included in a liquid detergent. A liquid detergent may contain from 0-30% organic solvent. A liquid detergent may even be non-aqueous, or substantially non-aqueous, wherein the water content is below 15%, preferably below 10%, more preferably below 6%, more preferably below 4%, more preferably below 2%, and most preferably below 1%.

Detergent ingredients can be separated physically from each other by compartments in water dissolvable pouches. Thereby negative storage interaction between components can be avoided. Different dissolution profiles of each of the compartments can also give rise to delayed dissolution of selected components in the wash solution.

The detergent composition may take the form of a unit dose product. A unit dose product is the packaging of a single dose in a non-reusable container. It is increasingly used in detergents for laundry and dish wash. A detergent unit dose product is the packaging (*e.g.*, in a pouch made from a water soluble film) of the amount of detergent used for a single wash.

Pouches can be of any form, shape and material which is suitable for holding the composition, *e.g.*, without allowing the release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxypropyl methyl cellulose (HPMC). Preferably the level of polymer in the film for example PVA is at least about 60%. Preferred average molecular weight will typically be about 20,000 to about 150,000. Films can also be a blend compositions comprising hydrolytically degradable and water soluble polymer blends such as polyactide and polyvinyl alcohol plus plasticizers like glycerol, ethylene glycerol, Propylene glycol, sorbitol and mixtures thereof. The pouches can comprise a solid laundry cleaning composition or part components and/or a liquid cleaning composition or part components separated by the water soluble film. The compartment for liquid components can be different in composition than compartments containing solids (see *e.g.*, US 2009/0011970).

The choice of detergent components may include, for textile care, the consideration of the type of textile to be cleaned, the type and/or degree of soiling, the temperature at which cleaning is to take place, and the formulation of the detergent product. Although components mentioned below are categorized by general header according to a particular functionality, this is not to be construed as a limitation, as a component may comprise additional functionalities as will be appreciated by the skilled artisan.

The choice of additional components is within the skill of the artisan and includes conventional ingredients, including the exemplary components set forth below.

In one embodiment of the present invention, the enzyme granule of the present invention may be added to a detergent composition in an amount corresponding to 0.001-200 mg of enzyme protein, such as 0.005-100 mg of enzyme protein, preferably 0.01-50 mg of enzyme protein, more preferably 0.05-20 mg of enzyme protein, even more preferably 0.1-10 mg of enzyme protein per liter of wash liquor.

## Surfactants

The detergent composition may comprise one or more surfactants, which may be anionic and/or cationic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 0.1% to 60% by weight, such as about 1% to about 40%, or about 3% to about 20%, or about 3% to about 10%. The surfactant(s) is chosen based on the desired cleaning application, and includes any conventional surfactant(s) known in the art. Any surfactant known in the art for use in detergents may be utilized.

When included therein the detergent will usually contain from about 1% to about 40% by weight, such as from about 5% to about 30%, including from about 5% to about 15%, or from about 20% to about 25% of an anionic surfactant. Examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or soap, and combinations thereof.

When included therein the detergent will usually contain from about 0.1% to about 10% by weight of a cationic surfactant. Examples of cationic surfactants include alklydimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, and combinations thereof.

When included therein the detergent will usually contain from about 0.2% to about 40% by weight of a non-ionic surfactant, for example from about 0.5% to about 30%, in particular from about 1% to about 20%, from about 3% to about 10%, such as from about 3% to about 5%, or from about 8% to about 12%. Examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxy alkyl fatty acid amides, or *N*-acyl *N*-alkyl derivatives of glucosamine (glucamides, GA, or fatty acid glucamide, FAGA), as well as products available under the trade names SPAN and TWEEN, and combinations

thereof.

When included therein the detergent will usually contain from about 0.1% to about 20% by weight of a semipolar surfactant. Examples of semipolar surfactants include amine oxides (AO) such as alkyldimethylamineoxide, *N*-(coco alkyl)-*N*,*N*-dimethylamine oxide and *N*-(tallow-alkyl)-*N*,*N*-bis(2-hydroxyethyl)amine oxide, fatty acid alkanolamides and ethoxylated fatty acid alkanolamides, and combinations thereof.

When included therein the detergent will usually contain from about 0.1% to about 10% by weight of a zwitterionic surfactant. Examples of zwitterionic surfactants include betaine, alkyldimethylbetaine, sulfobetaine, and combinations thereof.

Hydrotropes

A hydrotrope is a compound that solubilises hydrophobic compounds in aqueous solutions (or oppositely, polar substances in a non-polar environment). Typically, hydrotropes have both hydrophilic and a hydrophobic character (so-called amphiphilic properties as known from surfactants); however the molecular structure of hydrotropes generally do not favor spontaneous self-aggregation, see for example review by Hodgdon and Kaler (2007), Current Opinion in Colloid & Interface Science 12: 121-128. Hydrotropes do not display a critical concentration above which self-aggregation occurs as found for surfactants and lipids forming miceller, lamellar or other well defined meso-phases. Instead, many hydrotropes show a continuous-type aggregation process where the sizes of aggregates grow as concentration increases. However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in detergent compositions allow for example more concentrated formulations of surfactants (as in the process of compacting liquid detergents by removing water) without inducing undesired phenomena such as phase separation or high viscosity.

The detergent may contain 0-5% by weight, such as about 0.5 to about 5%, or about 3% to about 5%, of a hydrotrope. Any hydrotrope known in the art for use in detergents may be utilized. Examples of hydrotropes include sodium benzene sulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

Builders and Co-Builders

The detergent composition may contain about 0-65% by weight, such as about 5% to about 50% of a detergent builder or co-builder, or a mixture thereof. In a dish wash detergent, the level of builder is typically 40-65%, particularly 50-65%. The builder and/or co-builder may particularly be a chelating agent that forms water-soluble complexes with calcium and magnesium ions. Any builder and/or co-builder known in the art for use in laundry detergents may be utilized. Examples of builders include citrates, zeolites, diphosphates (pyrophosphates), triphosphates such as sodium triphosphate (STP or STPP), carbonates such as sodium carbonate, soluble silicates such as sodium metasilicate, layered silicates (*e.g.*, SKS-6 from Hoechst), ethanolamines such as 2-aminoethan-1-ol (MEA), diethanolamine (DEA, also known as iminodiethanol), triethanolamine (TEA, also known as 2,2',2"-nitrilotriethanol), and carboxymethyl inulin (CMI), and combinations thereof.

The detergent composition may also contain 0-50% by weight, such as about 5% to about 30%, of a detergent co-builder, or a mixture thereof. The detergent composition may include a co-builder alone, or in combination with a builder, for example a zeolite builder. Examples of co-builders include homopolymers of polyacrylates or copolymers thereof, such as poly(acrylic acid) (PAA) or copoly(acrylic acid/maleic acid) (PAA/PMA). Further examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-N,N'-disuccinic acid (EDDS), methylglycinediacetic acid (MGDA), glutamic acid-N,N-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), *N*-(2-hydroxyethyl)iminodiacetic acid (EDG), aspartic acid-*N*-monoacetic acid (ASMA), aspartic acid-*N*,*N*-diacetic acid (ASDA), aspartic acid-*N*-monopropionic acid (ASMP), iminodisuccinic acid (IDA), *N*-(2-sulfomethyl)-aspartic acid (SMAS), *N*-(2-sulfoethyl)-aspartic acid (SEAS), *N*-(2-sulfomethyl)-glutamic acid (SMGL), *N*-(2-sulfoethyl)-glutamic acid (SEGL), *N*-methyliminodiacetic acid (MIDA), α-alanine-*N*, *N*-diacetic acid (α-ALDA), serine-*N*, *N*-diacetic acid (SEDA), isoserine-*N*, *N*-diacetic acid (ISDA), phenylalanine-*N*, *N*-diacetic acid (PHDA), anthranilic acid-*N*, *N*-diacetic acid (ANDA), sulfanilic acid-*N*, *N*-diacetic acid (SLDA) , taurine-*N*, *N*-diacetic acid (TUDA) and sulfomethyl-*N*, *N*-diacetic acid (SMDA), *N*-(2-hydroxyethyl)-ethylidenediamine-*N*, *N'*, *N'*-triacetate (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, *e.g.*, WO 2009/102854, US 5,977,053.

Bleaching Systems

The detergent may contain 0-50% by weight of a bleaching system. Any bleaching system known in the art for use in laundry detergents may be utilized. Suitable bleaching system components include bleaching catalysts, photobleaches, bleach activators, sources of hydrogen peroxide such as sodium percarbonate and sodium perborates, preformed peracids and mixtures thereof. Suitable preformed peracids include, but are not limited to, peroxycarboxylic acids and salts, percarbonic acids and salts, perimidic acids and salts, peroxymonosulfuric acids and salts, for example, Oxone (R), and mixtures thereof. Examples of bleaching systems include peroxide-based bleaching systems, which may comprise, for example, an inorganic salt, including alkali metal salts such as sodium salts of perborate (usually mono- or tetra-hydrate), percarbonate, persulfate, perphosphate, persilicate salts, in combination with a peracid-forming bleach activator. The term bleach activator is meant herein as a compound which reacts with peroxygen bleach like hydrogen peroxide to form a peracid. The peracid thus formed constitutes the activated bleach. Suitable bleach activators to be used herein include those belonging to the class of esters amides, imides or anhydrides. Suitable examples are tetracetylethylene diamine (TAED), sodium 4-[(3,5,5-trimethylhexanoyl)oxy]benzene sulfonate (ISONOBS), diperoxy dodecanoic acid, 4-(dodecanoyloxy)benzenesulfonate (LOBS), 4-(decanoyloxy)benzenesulfonate, 4-(decanoyloxy)benzoate (DOBS), 4-(nonanoyloxy)-benzenesulfonate (NOBS), and/or those disclosed in WO 98/17767. A particular family of bleach activators of interest was disclosed in EP624154 and particulary preferred in that family is acetyl triethyl citrate (ATC). ATC or a short chain triglyceride like triacetin has the advantage that it is environmental friendly as it eventually degrades into citric acid and alcohol. Furthermore acetyl triethyl citrate and triacetin has a good hydrolytical stability in the product upon storage and it is an efficient bleach activator. Finally ATC provides a good building capacity to the laundry additive. Alternatively, the bleaching system may comprise peroxyacids of, for example, the amide, imide, or sulfone type. The bleaching system may also comprise peracids such as 6-(phthalimido)peroxyhexanoic acid (PAP). The bleaching system may also include a bleach catalyst. In some embodiments the bleach component may be an organic catalyst selected from the group consisting of organic catalysts having the following formulae:

(i) 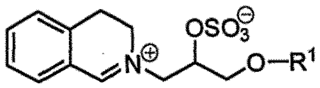

(ii) 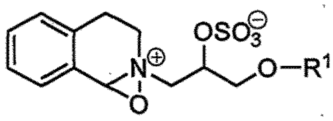

and mixtures thereof; wherein each $R^1$ is independently a branched alkyl group containing from 9 to 24 carbons or linear alkyl group containing from 11 to 24 carbons, preferably each $R^1$ is independently a branched alkyl group containing from 9 to 18 carbons or linear alkyl group containing from 11 to 18 carbons, more preferably each $R^1$ is independently selected from the group consisting of 2-propylheptyl, 2-butyloctyl, 2-pentylnonyl, 2-hexyldecyl, n-dodecyl, n-tetradecyl, n-hexadecyl, n-octadecyl, iso-nonyl, iso-decyl, iso-tridecyl and iso-pentadecyl. Other exemplary bleaching systems are described, *e.g.*, in WO 2007/087258, WO 2007/087244, WO 2007/087259 and WO 2007/087242. Suitable photobleaches may for example be sulfonated zinc phthalocyanine.

Polymers

The detergent may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide anti redeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-N-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, *e.g.*, WO 2006/130575 and US 5,955,415. Salts of the above-mentioned polymers are also contemplated.

Fabric hueing agents

The detergent compositions of the present invention may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO 2005/03274, WO 2005/03275, WO 2005/03276 and EP 1876226 (hereby incorporated by reference). The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, *e.g.*, WO 2007/087257 and WO 2007/087243.

Detergent enzyme(s)

The detergent additive as well as the detergent composition may comprise one or more (additional) enzymes, such as those mentioned above under the heading "Enzyme".

In general the properties of the selected enzyme(s) should be compatible with the selected detergent, (*i.e.,* pH-optimum, compatibility with other enzymatic and non-enzymatic ingredients, etc.), and the enzyme(s) should be present in effective amounts.

The detergent enzyme(s) may be included in a detergent composition by adding separate additives containing one or more enzymes, or by adding a combined additive comprising all of these enzymes. A detergent additive of the invention, *i.e.,* a separate additive or a combined additive, can be formulated, for example, as a granulate, liquid, slurry, etc. Preferred detergent additive formulations are granulates, in particular non-dusting granulates, liquids, in particular stabilized liquids, or slurries.

The detergent enzyme(s) may be included in a detergent composition by adding separate additives containing one or more enzymes, or by adding a combined additive comprising all of these enzymes. A detergent additive of the invention, *i.e.,* a separate additive or a combined additive is formulated as a granule of the invention.

Adjunct materials

Any detergent components known in the art for use in laundry detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in laundry detergents may be utilized. The choice of such ingredients is well within the skill of the artisan.

Dispersants - The detergent compositions of the present invention can also contain dispersants. In particular powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

Dye Transfer Inhibiting Agents - The detergent compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and *N*-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

Fluorescent whitening agent - The detergent compositions of the present invention will preferably also contain a fluorescent whitening agent. Fluorescent whitening agents, also referred to as optical brighteners, optical brightening agents, or fluorescent brightening agents, are dyes that absorb light in the ultraviolet and violet region (usually 340-370 nm) of the electromagnetic spectrum, and re-emit light in the blue region (typically 420-470 nm). These agents are often

used to enhance the appearance of color of fabric and paper, causing a whitening effect, making materials look less yellow by increasing the overall amount of blue light reflected.

Preferred fluorescent agents are selected from the classes, distyrylbiphenyls, triazinylaminostilbenes, bis(1,2,3-triazol-2-yl)stilbenes, bis(benzo[b]furan-2-yl)biphenyls, 1,3-diphenyl-2-pyrazolines, thiophenediyl benzoxazole, and courmarins. The fluorescent agent is preferably sulfonated.

Preferred classes of fluorescent agent are: di-styryl biphenyl compounds, e.g., Tinopal™ CBS-X; di-amine stilbene di-sulphonic acid compounds, *e.g.*, Tinopal DMS-X and Blankophor™ HRH; pyrazoline compounds, *e.g.*, Blankophor SN; and thiophenediyl benzoxazole compounds, *e.g.*, Tinopal OB.

Other fluorescent whitening agents suitable for use in detergent compositions include those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino) stilbene-2.2'-disulfonate, 4,4'-bis-(2-anilino-4-(*N*-methyl-*N*-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2*H*-naphtho[1,2-*d*][1,2,3]triazol-2-yl)-2-[(*E*)-2-phenylvinyl]benzenesulfonate. Preferred fluorescent whitening agents are Tinopal DMS and Tinopal CBS and Tinopal OB, available from BASF. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Tinopal OB is 2,5-thiophenediylbis(5-tert-butyl-1,3-benzoxazole). Another preferred fluorescent whitening agent is the commercially available Parawhite KX, supplied by Paramount Minerals and Chemicals, Mumbai, India. Other fluorescers suitable for use in the invention include the 1-3-diaryl pyrazolines and the 7-alkylaminocoumarins.

Suitable fluorescent brightener levels include lower levels of from about 0.01, from 0.05, from about 0.1 or even from about 0.2 wt % to upper levels of 0.5 or even 0.75 wt%. Preferably, the brightener is at a level of about 0.01% to about 0.5%.

Soil release polymers - The detergent compositions of the present invention may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523 (hereby incorporated by reference). Furthermore random graft co-polymers are suitable soil release polymers. Suitable graft co-polymers are described in more detail in WO 2007/138054, WO 2006/108856 and WO 2006/113314 (hereby incorporated by reference). Other soil release polymers are substituted polysaccharide structures especially substituted cellulosic structures such as modified cellulose deriviatives such as those described in EP 1867808 or WO 2003/040279 (both are hereby incorporated by reference). Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

Anti-redeposition agents - The detergent compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

Rheology Modifiers are structurants or thickeners, as distinct from viscosity reducing agents. The rheology modifiers are selected from the group consisting of non-polymeric crystalline, hydroxy-functional materials, polymeric rheology modifiers which impart shear thinning characteristics to the aqueous liquid matrix of a liquid detergent composition. The rheology and viscosity of the detergent can be modified and adjusted by methods known in the art, for example as shown in EP 2169040.

Other suitable adjunct materials include, but are not limited to, anti-shrink agents, anti-wrinkling agents, bactericides, binders, carriers, dyes, enzyme stabilizers, fabric softeners, fillers, foam regulators, perfumes, pigments, sod suppressors, and solvents.

Laundry soap bars

The granule of the invention may be added to laundry soap bars and used for hand washing laundry, fabrics and/or textiles. The term laundry soap bar includes laundry bars, soap bars, combo bars, syndet bars and detergent bars.

The types of bar usually differ in the type of surfactant they contain, and the term laundry soap bar includes those containing soaps from fatty acids and/or synthetic soaps. The laundry soap bar has a physical form which is solid and not a liquid, gel or a powder at room temperature. The term solid is defined as a physical form which does not significantly change over time, *i.e.,* if a solid object (*e.g.,* laundry soap bar) is placed inside a container, the solid object does not change to fill the container it is placed in. The bar is a solid typically in bar form but can be in other solid shapes such as round or oval.

The laundry soap bar may contain one or more additional enzymes, protease inhibitors such as peptide aldehydes (or hydrosulfite adduct or hemiacetal adduct), boric acid, borate, borax and/or phenylboronic acid derivatives such as 4-formylphenylboronic acid, one or more soaps or synthetic surfactants, polyols such as glycerine, pH controlling compounds such as fatty acids, citric acid, acetic acid and/or formic acid, and/or a salt of a monovalent cation and an organic anion wherein the monovalent cation may be for example $Na^+$, $K^+$ or $NH_4^+$ and the organic anion may be for example formate, acetate, citrate or lactate such that the salt of a monovalent cation and an organic anion may be, for example, sodium formate.

The laundry soap bar may also contain complexing agents like EDTA and HEDP, perfumes and/or different type of fillers, surfactants, *e.g*., anionic synthetic surfactants, builders, polymeric soil release agents, detergent chelators, stabilizing agents, fillers, dyes, colorants, dye transfer inhibitors, alkoxylated polycarbonates, suds suppressers, structurants, binders, leaching agents, bleaching activators, clay soil removal agents, anti-redeposition agents, polymeric dispersing agents, brighteners, fabric softeners, perfumes and/or other compounds known in the art.

The laundry soap bar may be processed in conventional laundry soap bar making equipment such as but not limited to: mixers, plodders, *e.g*., a two stage vacuum plodder, extruders, cutters, logo-stampers, cooling tunnels and wrappers. The invention is not limited to preparing the laundry soap bars by any single method. The premix of the invention may be added to the soap at different stages of the process. For example, the premix containing a soap, an enzyme granule of the invention, optionally one or more additional enzymes, a protease inhibitor, and a salt of a monovalent cation and an organic anion may be prepared and and the mixture is then plodded. The enzyme and optional additional enzymes may be added at the same time as the protease inhibitor for example in liquid form. Besides the mixing step and the plodding step, the process may further comprise the steps of milling, extruding, cutting, stamping, cooling and/or wrapping.

**Animal Feed and Animal Feed Additives**

The enzyme granule of the invention may be added to and thus become a component of an animal feed, or an animal feed additive.

In one aspect, the animal feed or animal feed additive comprises the enzyme granule of the invention, and one or more components selected from the group consisting of vitamins, minerals and amino acids.

In an embodiment, the animal feed or animal feed additive further comprises one or more additional enzymes selected from the group consisting of acetylxylan esterase, acylglycerol lipase, amylase, alpha-amylase, beta-amylase, arabinofuranosidase, cellobiohydrolases, cellulase, feruloyl esterase, galactanase, alpha-galactosidase, beta-galactosidase, beta-glucanase, beta-glucosidase, lysophospholipase, lysozyme, alpha-mannosidase, beta-mannosidase (mannanase), phytase, phospholipase A1, phospholipase A2, phospholipase D, protease, pullulanase, pectinesterase, triacylglycerol lipase, xylanase, beta-xylosidase or any combination thereof.

In an embodiment, the animal feed or animal feed additive comprises one or more microbes selected from the group consisting of *Bacillus subtilis, Bacillus licheniformis, Bacillus amyloliquefaciens, Bacillus cereus, Bacillus pumilus, Bacillus polymyxa, Bacillus megaterium, Bacillus coagulans, Bacillus circulans, Bifidobacterium bifidum, Bifidobacterium animalis, Bifidobacterium sp., Carnobacterium sp., Clostridium butyricum, Clostridium sp., Enterococcus faecium, Enterococcus sp., Lactobacillus sp., Lactobacillus acidophilus, Lactobacillus farciminus, Lactobacillus rhamnosus, Lactobacillus reuteri, Lactobacillus salivarius, Lactococcus lactis, Lactococcus sp., Leuconostoc sp., Megasphaera elsdenii, Megasphaera sp., Pediococcus acidilactici, Pediococcus sp., Propionibacterium thoenii, Propionibacterium sp.* and *Streptococcus sp.* or any combination thereof.

In one embodiment, the invention relates to an animal feed comprising the granule as described herein and plant based material. In one embodiment, the invention relates to an animal feed comprising the animal feed additive as described herein and plant based material. Animal feed compositions or diets have a relatively high content of protein. Poultry and pig diets can be characterised as indicated in Table B of WO 01/58275, columns 2-3. Fish diets can be characterised as indicated in column 4 of this Table B. Furthermore, such fish diets usually have a crude fat content of 200-310 g/kg.

An animal feed composition according to the invention has a crude protein content of 50-800 g/kg, and furthermore comprises an enzyme granule as claimed herein.

Furthermore, or in the alternative (to the crude protein content indicated above), the animal feed composition of the invention has a content of metabolisable energy of 10-30 MJ/kg; and/or a content of calcium of 0.1-200 g/kg; and/or a content of available phosphorus of 0.1-200 g/kg; and/or a content of methionine of 0.1-100 g/kg; and/or a content of methionine plus cysteine of 0.1-150 g/kg; and/or a content of lysine of 0.5-50 g/kg.

In particular embodiments, the content of metabolisable energy, crude protein, calcium, phosphorus, methionine, methionine plus cysteine, and/or lysine is within any one of ranges 2, 3, 4 or 5 in Table B of WO 01/58275 (R. 2-5).
Crude protein is calculated as nitrogen (N) multiplied by a factor 6.25, i.e. Crude protein (g/kg)= N (g/kg) x 6.25. The nitrogen content is determined by the Kjeldahl method (A.O.A.C., 1984, Official Methods of Analysis 14th ed., Association of Official Analytical Chemists, Washington DC).
Metabolisable energy can be calculated on the basis of the NRC publication Nutrient requirements in swine, ninth revised edition 1988, subcommittee on swine nutrition, committee on animal nutrition, board of agriculture, national research council. National Academy Press, Washington, D.C., pp. 2-6, and the European Table of Energy Values for Poultry Feed-stuffs, Spelderholt centre for poultry research and extension, 7361 DA Beekbergen, The Netherlands. Grafisch bedrijf Ponsen & looijen bv, Wageningen. ISBN 90-71463-12-5.
The dietary content of calcium, available phosphorus and amino acids in complete animal diets is calculated on the basis of feed tables such as Veevoedertabel 1997, gegevens over chemische samenstelling, verteerbaarheid en voederwaarde van voedermiddelen, Central Veevoederbureau, Runderweg 6, 8219 pk Lelystad. ISBN 90-72839-13-7.
In a particular embodiment, the animal feed composition of the invention contains at least one vegetable protein as defined above.
The animal feed composition of the invention may also contain animal protein, such as Meat and Bone Meal, Feather meal, and/or Fish Meal, typically in an amount of 0-25%. The animal feed composition of the invention may also comprise Dried Distillers Grains with Solubles (DDGS), typically in amounts of 0-30%.
In still further particular embodiments, the animal feed composition of the invention contains 0-80% maize; and/or 0-80% sorghum; and/or 0-70% wheat; and/or 0-70% Barley; and/or 0-30% oats; and/or 0-40% soybean meal; and/or 0-25% fish meal; and/or 0-25% meat and bone meal; and/or 0-20% whey.
The animal feed may comprise vegetable proteins. In particular embodiments, the protein content of the vegetable proteins is at least 10, 20, 30, 40, 50, 60, 70, 80, or 90% (w/w). Vegetable proteins may be derived from vegetable protein sources, such as legumes and cereals, for example, materials from plants of the families *Fabaceae (Leguminosae), Cruciferaceae, Chenopodiaceae,* and *Poaceae,* such as soy bean meal, lupin meal, rapeseed meal, and combinations thereof.
In a particular embodiment, the vegetable protein source is material from one or more plants of the family *Fabaceae, e.g.,* soybean, lupine, pea, or bean. In another particular embodiment, the vegetable protein source is material from one or more plants of the family *Chenopodiaceae,* e.g. beet, sugar beet, spinach or quinoa. Other examples of vegetable protein sources are rapeseed, and cabbage. In another particular embodiment, soybean is a preferred vegetable protein source. Other examples of vegetable protein sources are cereals such as barley, wheat, rye, oat, maize (corn), rice, and sorghum.
Animal diets can e.g. be manufactured as mash feed (non-pelleted) or pelleted feed. Typically, the milled feed-stuffs are mixed and sufficient amounts of essential vitamins and minerals are added according to the specifications for the species in question.

**Food Additive and Food Processing Aid**

The enzyme granule of the invention may be added to and thus become a component of a food additive or food processing aid, which are used in the food industry for the preparation of processed food products for human consumption.

**Compositions, methods and uses**

In a first aspect, the invention provides an enzyme granule comprising an enzyme and one or more spore(s), preferably bacterial spore(s). Preferably, the enzyme is a protease, lipase, cutinase, amylase, carbohydrase, cellulase, pectinase, pectate lyase, mannanase, arabinase, galactanase, xylanase, DNase, oxidase, laccase, peroxidase, or perhydrolase.
The enzyme granule comprises a core and a coating, where the core includes the enzyme and the coating includes the spore(s).
In an embodiment, the enzyme granule contains the enzyme in an amount of more than 0.5% w/w and less than 50% w/w active enzyme protein.
In an embodiment, the enzyme granule contains the spore(s) in a quantity between $1x10^2$ and $1x10^{12}$ CFU/g of the granule. Preferably, the one or more spore(s) is derived from spore forming bacterial strain(s). More preferably, the spore(s) are *Bacillus* endospore(s) derived from *a Bacillus* strain.
In an embodiment, the enzyme granule comprises a salt coating.
In another aspect, the invention provides a method for preparing the enzyme granule of the invention, as described above, which comprises granulating a blend which comprises the enzyme, followed by applying a coating which comprises the one or more spore(s).

In another aspect, the invention provides a detergent composition (e.g., a laundry or automatic dishwash detergent), comprising a surfactant and/or a detergent builder, and the enzyme granule of the invention, as described above. Preferably, detergent composition is a powder detergent composition.
The present invention is further described by the following examples.

## EXAMPLES

Chemicals were commercial products of at least reagent grade.

### Example 1A

A powder composition of 10% cellulose, 5% dextrin, 8% calcium carbonate and 77% sodium sulfate was granulated in a 50 L Lödige high shear granulator according to US 4,106,991 using about 15% water on dry basis. The product was dried in a pilot scale fluid bed and subsequently screened into the size range 300 μm - 1180 μm.
4000 g of above granules were loaded into a pilot scale fluid bed (Glatt Procel) equipped with a two-fluid top-spray nozzle. The air flow rate was adjusted to 120 $m^3$/h and the inlet temperature was set to 80°C.
A liquid composition consisting of 20 g HPMC and 20 g PEG 4000 dissolved into 400 g water was prepared. To this composition 1.15 g of a *Bacillus* endospore containing concentrate was added. The spore count in this concentrate was measured to be 4.6 x $10^{10}$ CFU/g.
The spore containing liquid composition was sprayed onto the fluidizing enzyme granules through the two-fluid nozzle using an atomization pressure of 3 bar. The spray rate was adjusted to about 30 g/min, which resulted in a product temperature of 45°C - 50°C and a relative humidity in the exhaust gas of about 30%.
After spraying the product was dried for 5 minutes and cooled for 15 minutes before discharge.

### Example 1B

The process in Example 1A was repeated except for the amount of spore concentrate added was increased from 1.15 g to 3.45 g.

### Example 1C

The process in Example 1A was repeated except for the amount of spore concentrate added was increased from 1.15 g to 11.5 g.

### Example 2A

4000 g of a commercial available enzyme (protease) granule manufactured by the process described in US 4,106,991 (Savinase) was loaded into a pilot scale fluid bed (Glatt Procel) equipped with a two-fluid top-spray nozzle. The air flow rate was adjusted to 120 $m^3$/h and the inlet temperature was set to 80°C.
A liquid composition consisting of 20 g HPMC and 20 g PEG 4000 dissolved into 400 g water was prepared. To this composition 1.15 g of a *Bacillus* endospore containing concentrate was added. The spore count in this concentrate was measured to be 4.6 x $10^{10}$ CFU/g.
The spore containing liquid composition was sprayed onto the fluidizing enzyme granules through the two-fluid nozzle using an atomization pressure of 3 bar. The spray rate was adjusted to about 30 g/min, which resulted in a product temperature of 45°C - 50°C and a relative humidity in the exhaust gas of about 30%.
After spraying the product was dried for 5 minutes and cooled for 15 minutes before discharge.

### Example 2B

The process in Example 2A was repeated except for the amount of spore concentrate added was increased from 1.15 g to 3.45 g.

### Example 2C

The process in Example 2A was repeated except for the amount of spore concentrate added was increased from 1.15 g to 11.5 g.

Table 1. The products manufactured in Examples 1A, 1B, 1C, and in Examples 2A, 2B, 2C were analyzed for viable spore counts.

| Example # | Enzyme in granule | Spore concentrate added (g/kg granule) | Viable spore count (CFU/g) | Viable spore yield (%) |
|---|---|---|---|---|
| 1A | No | 1.15 | $2.7 \times 10^6$ | 14 |
| 1B | No | 3.45 | $6.47 \times 10^6$ | 14 |
| 1C | No | 11.5 | $2.44 \times 10^7$ | 14 |
| 2A | Yes | 1.15 | $1.12 \times 10^7$ | 84 |
| 2B | Yes | 3.45 | $2.37 \times 10^7$ | 59 |
| 2C | Yes | 11.5 | $5.00 \times 10^7$ | 38 |

Surprisingly, the spore yields are better for the enzyme containing granules (Examples 2A, 2B, 2C) than for the granules without enzyme (Examples 1A, 1B, 1C). Normally it would be expected that the presence of an active enzyme - especially a protease - could harm the viability of the bacterial spores.

The possibility to use the same granule for delivering the enzymes as well as the spores is very attractive from both a technical, economic and environmental point of view because the requirements for raw materials, processing time and equipment are reduced.

## Claims

**1.** An enzyme granule comprising a core and a coating, wherein the core comprises an enzyme and the coating comprises one or more spores.

**2.** The granule of claim 1, wherein the enzyme is a protease, a lipase, a cutinase, an amylase, a carbohydrase, a cellulase, a pectinase, a pectate lyase, a mannanase, an arabinase, a galactanase, a xylanase, an oxidase, a laccase, a peroxidase, or a perhydrolase.

**3.** The granule of claim 1 or 2, which contains at least one enzyme in an amount of more than 0.5% w/w and less than 50% w/w active enzyme protein.

**4.** The granule of any of claims 1-3, which comprises a salt coating.

**5.** The granule of any of claims 1-4, wherein the spore(s) are bacterial spore(s).

**6.** The granule of any of claims 1-5, wherein the spore(s) are derived from spore forming bacterial strain(s).

**7.** The granule of any of claims 1-6, wherein the spore(s) are *Bacillus* endospore(s) derived from *a Bacillus* strain.

**8.** The granule of any of claims 1-7, which contains the spore(s) in a quantity between $1 \times 10^2$ and $1 \times 10^{12}$ CFU/g of the granule.

**9.** A method for preparing the enzyme granule of any of claims 1-8, which comprises granulating a blend which comprises the enzyme and/or the spore(s), followed by applying a coating which comprises the enzyme or the spore(s) if they are not included in the granulated blend.

**10.** A detergent composition, comprising a surfactant and/or a detergent builder and the enzyme granule of any of claims 1-8.

**11.** An animal feed or animal feed additive comprising the enzyme granule of any of claims 1-8, and plant based material.

## Patentansprüche

1. Enzymkörnchen, umfassend einen Kern und eine Beschichtung, wobei der Kern ein Enzym umfasst und die Beschichtung eine oder mehrere Sporen umfasst.

2. Körnchen nach Anspruch 1, wobei das Enzym eine Protease, eine Lipase, eine Cutinase, eine Amylase, eine Carbohydrase, eine Cellulase, eine Pectinase, eine Pectatlyase, eine Mannanase, eine Arabinase, eine Galactanase, eine Xylanase, eine Oxidase, eine Laccase, eine Peroxidase oder eine Perhydrolase ist.

3. Körnchen nach Anspruch 1 oder 2, das mindestens ein Enzym in einer Menge von mehr als 0,5% Gew./Gew. und weniger als 50% Gew./Gew. aktives Enzymprotein enthält.

4. Körnchen nach einem beliebigen der Ansprüche 1-3, das eine Salzbeschichtung umfasst.

5. Körnchen nach einem beliebigen der Ansprüche 1-4, wobei die Spore(n) Bakterienspore(n) sind.

6. Körnchen nach einem beliebigen der Ansprüche 1-5, wobei die Spore(n) aus einem sporenbildenden Bakterienstamm/ sporenbildenden Bakterienstämmen abgeleitet sind.

7. Körnchen nach einem beliebigen der Ansprüche 1-6, wobei die Spore(n) Bacillus-Endospore(n) sind, die aus einem Bacillus-Stamm abgeleitet sind.

8. Körnchen nach einem beliebigen der Ansprüche 1-7, das die Spore(n) in einer Menge zwischen $1x10^2$ und $1x10^{12}$ KBE/g des Körnchens enthält.

9. Verfahren zum Herstellen des Enzymkörnchens gemäß einem beliebigen der Ansprüche 1-8, das Granulieren einer Mischung umfasst, die das Enzym und/oder die Spore(n) umfasst, gefolgt von Aufbringen einer Beschichtung, die das Enzym oder die Spore(n) umfasst, wenn sie nicht in der granulierten Mischung eingeschlossen sind.

10. Detergenszusammensetzung, die ein oberflächenaktives Mittel und/oder einen Detergensgerüststoff und das Enzymkörnchen gemäß einem beliebigen der Ansprüche 1-8 umfasst.

11. Tierfutter oder Tierfutterzusatz, umfassend das Enzymkörnchen gemäß einem beliebigen der Ansprüche 1-8 und Material auf Pflanzenbasis.

## Revendications

1. Granule d'enzyme comprenant un noyau et un enrobage, dans lequel le noyau comprend une enzyme et l'enrobage comprend une ou plusieurs spores.

2. Granule selon la revendication 1, dans lequel l'enzyme est une protéase, une lipase, une cutinase, une amylase, une carbohydrase, une cellulase, une pectinase, une pectate lyase, une mannanase, une arabinase, une galactanase, une xylanase, une oxydase, une laccase, une peroxydase, ou une perhydrolase.

3. Granule selon la revendication 1 ou 2, qui contient au moins une enzyme en une quantité supérieure à 0,5 % p/p et inférieure à 50 % p/p de protéine enzymatique active.

4. Granule selon l'une quelconque des revendications 1 à 3, qui comprend un enrobage de sel.

5. Granule selon l'une quelconque des revendications 1 à 4, dans lequel la ou les spores sont une ou plusieurs spores bactériennes.

6. Granule selon l'une quelconque des revendications 1 à 5, dans lequel la ou les spores sont dérivées d'une ou plusieurs souches bactériennes formant des spores.

7. Granule selon l'une quelconque des revendications 1 à 6, dans lequel la ou les spores sont une ou plusieurs endospores de *Bacillus* dérivées d'une souche de *Bacillus.*

**8.** Granule selon l'une quelconque des revendications 1 à 7, qui contient la ou les spores en une quantité comprise entre 1 x $10^2$ et 1 x $10^{12}$ UFC/g du granule.

**9.** Méthode pour préparer le granule d'enzyme de l'une quelconque des revendications 1 à 8, qui comprend la granulation d'un mélange qui comprend l'enzyme et/ou la ou les spores, suivie de l'application d'un enrobage qui comprend l'enzyme ou la ou les spores si elles ne sont pas incluses dans le mélange granulé.

**10.** Composition détergente comprenant un tensioactif et/ou un adjuvant de détergent et le granule d'enzyme de l'une quelconque des revendications 1 à 8.

**11.** Aliment pour animaux ou additif pour aliment pour animaux comprenant le granule d'enzyme de l'une quelconque des revendications 1 à 8, et un matériau d'origine végétale.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012112718 A
- WO 2011018509 A
- WO 2006121596 A
- EP 356240 A
- WO 9723606 A
- WO 9739116 A
- US 4016040 A
- US 4713245 A
- US 4106991 A
- EP 170360 A
- EP 304332 A
- EP 304331 A
- WO 9009440 A
- WO 9009428 A
- WO 9307263 A
- WO 0001793 A
- WO 2006034710 A
- WO 9932595 A
- WO 8906279 A
- WO 9318140 A
- WO 98020115 A
- WO 0144452 A
- WO 0158275 A
- WO 0158276 A
- WO 03006602 A
- WO 04099401 A
- WO 9219729 A
- WO 96034946 A
- WO 9820115 A
- WO 9820116 A
- WO 99011768 A
- WO 0403186 A
- WO 04041979 A
- WO 07006305 A
- WO 11036263 A
- WO 11036264 A
- WO 2004067737 A
- US 5352604 A
- US 6124127 A
- WO 99027083 A
- WO 99027084 A
- WO 02006442 A
- WO 02092741 A
- WO 03095638 A
- WO 99064619 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- DK 9800299 W
- EP 258068 A
- EP 305216 A
- WO 9613580 A
- EP 218272 A
- EP 331376 A
- GB 1372034 A
- WO 9506720 A
- WO 9627002 A
- WO 9612012 A
- JP 64744992 B
- WO 9116422 A
- WO 9205249 A
- WO 9401541 A
- EP 407225 A
- EP 260105 A
- WO 9535381 A
- WO 9600292 A
- WO 9530744 A
- WO 9425578 A
- WO 9514783 A
- WO 9522615 A
- WO 9704079 A
- WO 9707202 A
- WO 00060063 A
- WO 2007087508 A
- WO 2009109500 A
- GB 1296839 A
- WO 9510603 A
- WO 9402597 A
- WO 9418314 A
- WO 9743424 A
- WO 99019467 A
- WO 02010355 A
- WO 2006066594 A
- WO 96023873 A

- WO 08153815 A
- WO 0166712 A
- WO 09061380 A
- WO 2011098531 A
- WO 2013001078 A
- WO 2013001087 A
- WO 2011098579 A
- WO 2014087011 A
- WO 2005056782 A
- WO 2008063400 A
- US 2008145353 A
- US 2007167344 A
- EP 179486 A
- WO 9324618 A
- WO 9510602 A
- WO 9815257 A
- WO 9527046 A
- WO 9704102 A
- WO 0179459 A
- WO 0179458 A
- WO 0179461 A
- WO 0179460 A
- WO 9201046 A
- JP 2238885 A
- WO 9708325 A
- WO 9533836 A
- WO 2008051491 A
- WO 9517413 A
- WO 9522625 A
- US 5223409 A
- WO 9206204 A
- US 20090011970 A
- WO 2009102854 A
- US 5977053 A
- WO 9817767 A
- EP 624154 A
- WO 2007087258 A
- WO 2007087244 A
- WO 2007087259 A
- WO 2007087242 A
- WO 2006130575 A
- US 5955415 A
- WO 200503274 A
- WO 200503275 A
- WO 200503276 A
- EP 1876226 A
- WO 2007087257 A
- WO 2007087243 A
- WO 2009087523 A
- WO 2007138054 A
- WO 2006108856 A
- WO 2006113314 A
- EP 1867808 A
- WO 2003040279 A
- EP 2169040 A

**Non-patent literature cited in the description**


- **C. E. CAPES**. Handbook of Powder Technology; Particle size enlargement. Elsevier, 1980, vol. 1
- Powdered detergents. Surfactant Science Series. Marcel Dekker, 1998, vol. 71, 140-142
- Principles of Powder Technology. John Wiley & Sons, 1990
- **DONNELLAN, J. E.** ; **NAGS, E. H.** ; **LEVINSON, H. S.** Chemically defined, synthetic media for sporulation and for germination and growth of Bacillus subtilis. *Journal of Bacteriologyl*, 1964, vol. 87 (2), 332-336
- **CHEN, Z.** ; **LI, Q.** ; **LIU, H.** ; **YU, N.** ; **XIE, T.** ; **YANG, M.** ; **SHEN, P.** ; **CHEN, X.** Greater enhancement of Bacillus subtilis spore yields in submerged cultures by optimization of medium composition through statistical experimental designs. *Appl. Microbiol. Biotechnol.*, 2010, vol. 85, 1353-1360
- **WHITE** ; **HANDLER** ; **SMITH**. Principles of Biochemistry. McGraw-Hill Book Company, 1973, 271-272
- **SIEZEN et al.** *Protein Engng*, 1991, vol. 4, 719-737
- **SIEZEN et al.** *Protein Science*, 1997, vol. 6, 501-523
- **DARTOIS et al.** *Biochemica et Biophysica Acta*, 1993, vol. 1131, 253-360
- **H. NEURATH** ; **R.L. HILL**. The Proteins. Academic Press, 1979
- **CUNNINGHAM** ; **WELLS**. *Science*, 1989, vol. 244, 1081-1085
- **HILTON et al.** *J. Biol. Chem.*, 1996, vol. 271, 4699-4708
- **DE VOS et al.** *Science*, 1992, vol. 255, 306-312
- **SMITH et al.** *J. Mol. Biol.*, 1992, vol. 224, 899-904
- **WLODAVER et al.** *FEBS Lett.*, 1992, vol. 309, 59-64
- **REIDHAAR-OLSON** ; **SAUER**. *Science*, 1988, vol. 241, 53-57
- **BOWIE** ; **SAUER**. *Proc. Natl. Acad. Sci. USA*, 1989, vol. 86, 2152-2156
- **LOWMAN et al.** *Biochemistry*, 1991, vol. 30, 10832-10837
- **DERBYSHIRE et al.** *Gene*, 1986, vol. 46, 145
- **NER et al.** *DNA*, 1988, vol. 7, 127
- **NEEDLEMAN** ; **WUNSCH**. *J. Mol. Biol.*, 1970, vol. 48, 443-453

- **RICE et al.** EMBOSS: The European Molecular Biology Open Software Suite. *Trends Genet.*, 2000, vol. 16, 276-277
- **HODGDON** ; **KALER**. *Current Opinion in Colloid & Interface Science*, 2007, vol. 12, 121-128
- Powdered Detergents. Surfactant science series. Marcel Dekker, Inc., vol. 71
- Official Methods of Analysis. Association of Official Analytical Chemists, 1984
- NRC publication Nutrient requirements in swine. National Academy Press, 1988, 2-6
- **GRAFISCH BEDRIJF PONSEN** ; **LOOIJEN BV, WAGENINGEN**. European Table of Energy Values for Poultry Feed-stuffs. Spelderholt centre for poultry research and extension
- gegevens over chemische samenstelling, verteerbaarheid en voederwaarde van voedermiddelen. Veevoedertabel. Central Veevoederbureau, 1997